## Europäisches Patentamt
## European Patent Office
## Office européen des brevets

(19)

(11) Veröffentlichungsnummer : **0 027 160**
**B1**

(12)

# EUROPÄISCHE PATENTSCHRIFT

(45) Veröffentlichungstag der Patentschrift :
26.09.84

(51) Int. Cl.³ : **A 61 F 1/03**

(21) Anmeldenummer : **80104099.9**

(22) Anmeldetag : **15.07.80**

(54) **Hüftgelenkprothese.**

(30) Priorität : **11.10.79 CH 9216/79**

(43) Veröffentlichungstag der Anmeldung :
**22.04.81 Patentblatt 81/16**

(45) Bekanntmachung des Hinweises auf die Patenterteilung : **26.09.84 Patentblatt 84/39**

(84) Benannte Vertragsstaaten :
**AT BE DE FR GB IT NL SE**

(56) Entgegenhaltungen :
**DE-A- 2 246 940
DE-A- 2 324 865
DE-A- 2 537 807
DE-A- 2 547 790
US-A- 3 740 769
ORTHOPÄDE, Band 8, 1979, M.E. MÜLLER et al.
"Coxarthrose", Seiten 73,74
Prospekt 81 ter Fa. PROTEK Ltd., Switzerland**

(73) Patentinhaber : **GEBRÜDER SULZER AKTIENGE-
SELLSCHAFT
Zürcherstrasse 9
CH-8401 Winterthur (CH)
Protek AG
Stadtbachstrasse 64
CH-3001 Bern (CH)**

(72) Erfinder : **Niederer, Peter Gino
Reichenbachstrasse 6
CH-3052 Zollikofen (CH)**

(74) Vertreter : **Dipl.-Ing. H. Marsch Dipl.-Ing. K. Sparing
Dipl.-Phys.Dr. W.H. Röhl Patentanwälte
Rethelstrasse 123
D-4000 Düsseldorf (DE)**

## Beschreibung

Die Erfindung betrifft eine Hüftgelenkprothese mit einem geraden, blattartigen Schaft für die Verankerung durch Verkeilung und über ein Zementbett im Femur, wobei der Schaft sich vom distalen freien Ende aus zunächst symmetrisch zu einer Längsmittelachse des Blattes konisch erweitert, wobei ferner die Konusfläche der lateralen Schmalseite auf etwa 3/4 der Schafthöhe in eine zur Längsmittelachse geneigte Schrägfläche übergeht, während die mediale Schmalseite aus dem Konus heraus in einer stetig gekrümmten Kurve stufenlos in den Prothesenhals mündet und wobei schliesslich ein kragenartiger Ansatz die Blattseiten vom Prothesenhals trennt.

Aus der US-A-3,740,769 ist eine Prothesenkonstruktion bekannt, deren geradliniger Schaft einen in seiner Grundform runden Querschnitt hat. Unterhalb des Bogens, mit dem der Schaft medial in den Prothesenhals übergeht, ist ein vorspringender Ansatz vorgesehen, mit dem sich die Prothese auf dem kleinen Trochanter abstützt. Für einen Einsatz bei Patienten mit hypoplastischen Hüftgelenken, d. h. mit — im allgemeinen — von Geburt an deformierten und geschwächten Gelenken, bei denen der Femur zusätzlich zu seiner Deformation relativ schwach und dünn ist, ist diese Konstruktion nicht geeignet. Derar verformte Hüftgelenke sind aber das Einsatzgebiet für die Prothesen nach der vorliegenden Erfindung.

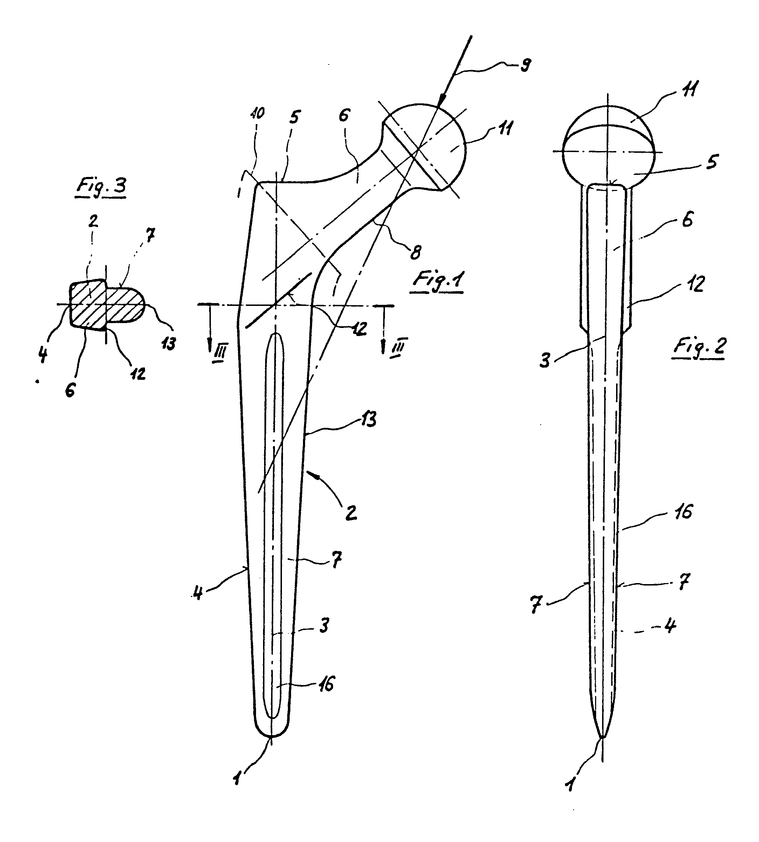

Hüftgelenkprothesen der eingangs genannten Art sind beispielsweise aus der Zeitschritt « Orthopäde » 8 (1979), Seite 74/74, insbesondere Abbildung 1, bekannt. Der sogenannte Geradschaft dieser Prothese hat die Aufgabe, sich in dem operativ auf seine Dimensionen abgestimmten Hohlraum der Markhöhle und einem, in diese eingefüllten Bett aus Knochenzement so zu verklemmen, dass das Zementbett oder der Zementköcher weitgehend von tragenden Funktionen entlastet wird ; die tragende Abstützung dieser Prothese erfolgt dabei in erster Linie durch Verklemmung entlang der medialen und lateralen Schmalseiten des Schaftes.

Bei dieser Prothesen-Konstruktion für « normale » Hüftgelenke verläuft der kragenartige Ansatz, der den Prothesenhals vom Schaft der Prothese trennt, so, dass er im wesentlichen senkrecht zur Prothesenhalsachse liegt. Der kragenartige Ansatz liegt dann im wesentlichen parallel zur Schnittfläche durch den Schenkelhals, so dass er sich auf dem Rand des den Knochen umgebenden Zementköchers zusätzlich abstützt, um ein Einsinken des Schaftes in den Knochenzement zu verhindern. Bekanntlich ergibt sich bei Hüftgelenkprothesen wegen der am Gelenkkopf angreifenden Belastung infolge des als « Hebel » wirkenden Prothesenhalses eine Drehmomentwirkung, durch welche das relativ dünne Blatt der diskutierten Prothesenkonstruktion relativ stark auf Biegung und Torsion beansprucht wird. Bei Patienten mit hypoplastischen Hüftgelenken ist der Femur im Bereich des Schenkelhalses und des Gelenkkopfes im allgemeinen verbildet und verkrüppelt und der Markraum ist oft äusserst eng. Prothesen der eingangs genannten Konstruktion für diese Krankheitsfälle sind daher relativ dünn und mit relativ schmalem Blatt versehen. Aufgrund des geschilderten Drehmomentes kann es daher in dem relativ dünnen und schmalen Blatt dieser Prothesen zu überelastischen plastischen Verformungen kommen. Aufgabe der Erfindung ist es, diese Schwierigkeiten zu beheben und ein plastisches Verformen des Prothesenblattes sowie dessen Verdrehung im Zement- und Knochenlager aufgrund der Drehmomentwirkung zu verhindern. Erfindungsgemäss wird diese Aufgabe dadurch gelöst, dass der auf die Blattseiten beschränkte kragenartige Ansatz mindestens annähernd in Richtung des Schenkelhalses verläuft.

Im Bereich der kritischen Belastungen ist bei der neuen Konstruktion der Prothesenschaft dadurch entsprechend verstärkt, so dass auch bei den dünnen und schmalen Schäften für Dysplasie-Patentien ein plastisches Verformen nicht mehr befürchtet werden muss. Ferner erlaubt die erfindungsgemässe Anordnung des Prothesenhalses, dass dieser im Bereich des medialen Calcarbogens direkt im Knochen abgestützt werden kann im Sinne einer weiteren Verminderung der erwähnten Drehmomentwirkung durch die Belastung. Darüberhinaus wird auch — ähnlich wie beim kragenartigen Ansatz der üblichen Prothesen — eine gute Abstützung der beschriebenen Prothese im Zement ermöglicht, darüberhinaus verhindert der kragenartige Ansatz ein Herausquellen des Knochenzementes beim Einsetzen der Prothese.

Die angestrebte Wirkung der neuen Konstruktion kann verbessert werden, wenn der kragenartigen Ansatz mindestens annähernd fluchtend mit der unteren Prothesenhalskante verläuft. Der Prothesenhals wird dadurch funktionell als Teil des Prothesenschaftes verstanden, da er — im Unterschied zu den üblichen Prothesen — einen Anteil der Abstützung im Knochen- und Zementlager übernimmt.

Im folgenden wird die Erfindung anhand von einem Ausführungsbeispiel im Zusammenhang mit der Zeichnung näher erläutert.

Figur 1 zeigt die neue Prothese in einer Aufsicht auf das Schaftblatt, während

Figur 2 eine Ansicht von Fig. 1 von links darstellt ;

Figur 3 ist der Schnitt III-III von Fig. 1.

Vom distalen Ende aus erweitert sich der blattartige Schaft 2 symmetrisch zu einer Längsmittelachse 3 konisch ; etwa auf 3/4 der Schafthöhe besitzt die laterale Schmalseite 4 des Schaftes 2 eine Unstetigkeit, an der der sich vom distalen Ende her erweiternde Konus in eine zur Längsmittelachse 3 hin verlaufende Schrägfläche übergeht. Diese endet in einer, mindestens nahe-

zu horizonalen Schulter 5, die den Uebergang zum Prothesenhals 6 bildet ; auf dessen freien Ende sitzt der Gelenkkopf 11, auf dem in Fig. 1 mit dem Pfeil 9 die Richtung der Hauptbelastung der Prothese angedeutet ist.

Etwas oberhalb der lateral gelegenen Unstetigkeit geht der sich erweiternde Konus der medialen Schmalseite 13 des Schaftblattes 7 in einen Kreisbogen über, der den Konus stufenlos mit der Unterkante 8 des Prothesenhalses 6 verbindet.

Die relativ dünnen und schmalen Schaftblätter 7 enden oben in einem kragenartigen Ansatz 12, der erfindungsgemäss etwa in Richtung parallel zur Schenkelhalsachse verläuft und den Uebergang zum dickeren Schenkelhals bildet. Die Höhe des kragenartigen Ansatzes 12 ist dabei so gewählt, dass er eine Verlängerung der Unterkante 8 des Prothesenhalses ist.

Bei einer richtig in ein Zementbett eingesetzten Prothese sind der kragenartige Ansatz 12 und der von ihm zur Schulter 5 hin verlaufende verlängerte Prothesenhals 6 praktisch völlig oder zumindest weitgehend im Zementbett versenkt, was in Fig. 1 durch die — etwa die obere Begrenzung des Zementbettes wiedergebende — strichelte Linie 10 angedeutet ist. Unabhängig davon, wo man den Drehpunkt für das auf Grund der exzentrischen Belastung auftretende Drehmoment am kragenartigen Ansatz 12 annehmen muss, wird dadurch — neben der verminderten Gefahr einer plastischen Verformung — eine bessere Verankerung des Schaftes im Zement- und Knochenlager erreicht.

Am distalen Ende 1 sind die Blattseiten 7, in denen Längsnuten 16 vorgesehen sind, durch einen kreisförmigen, von der lateralen Schmalseite 4 zur medialen Schmalseite 13 verlaufenden Bogen abgeschlossen, während die beiden Blattseiten 7 mit einem relativ grossen Radius in einer Spitze auslaufen (Fig. 12). Die Krümmung dieses Auslaufens ist dabei so gewählt, dass möglichst ein stetiger Uebergang des belastenden kraftflusses vom Schaft 2 auf den ihn umgebenden Zementköcher und weiter auf das — unter Umständen beim Einschlagen verdichtete — spongiose Knochengewebe erfolgt.

## Ansprüche

1. Hüftgelenkprothese mit einem geraden, blattartigen Schaft (2) für die Verankerung durch Verkeilung und über ein Zementbett im Femur, wobei der Schaft (2) sich vom distalen freien Ende aus zunächst symmetrisch zu einer Längsmittelachse (3) des Blattes konisch erweitert, wobei ferner die Konusfläche der lateralen Schmalseite (4) auf etwa 3/4 der Schafthöhe in eine zur Längsmittelachse (3) geneigte Schrägfläche übergeht, während die mediale Schmalseite (13) aus dem Konus heraus in einer stetig gekrümmten Kurve stufenlos in den Prothesenhals (6) mündet und wobei schliesslich ein kragenartiger Ansatz (12) die Blattseiten (7) vom Prothesenhals (6) trennt, dadurch gekennzeichnet, dass der auf die Blattseiten (7) beschränkte kragenartige Ansatz (12) mindestens annähernd in Richtung des Prothesennales (6) verläuft.

2. Hüftgelenkprothese nach Anspruch 1, dadurch gekennzeichnet, dass der kragenartige Ansatz (12) mindestens annähernd fluchtend mit der unteren Prothesenhalskante (8) verläuft.

## Claims

1. A hip joint prosthesis having a straight shank (2) after the style of a blade for anchoring by wedging and via a cement bed in the femur, the shank (2) widening conically, from the distal free end, initially symmetrically to a longitudinal central axis (3) of the blade, the conical surface of the lateral narrow side (4) merging, at about three-quarters of the shank height, into an oblique surface at an angle to the longitudinal central axis (3), while the medial narrow side (13) extends from the cone in a continuously radiused curve steplessly into the neck (6) of the prosthesis, and an extension (12) in the form of a collar separates the blade sides (7) from the prosthesis neck (6), characterised in that the collar-like extension (12) restricted to the blade sides (7) extends at least approximately in the direction of the prosthesis neck (6).

2. A hip joint prosthesis according to claim 1, characterised in that the collar-like extension (12) extends at least approximately in line with the bottom prosthesis neck edge (8).

## Revendications

1. Prothèse coxo-fémorale comportant une broche (2) rectiligne et en forme de spatule pour l'ancrage par coincement et par l'intermédiaire d'un lit de ciment dans le fémur, ladite broche (2) s'élargissant tronconiquement à partir de son extrémité libre distale, tout d'abord symétriquement par rapport à un axe médian longitudinal (3) de la spatule, la surface tronconique du petit côté latéral (4) se prolongeant, environ aux 3/4 de la hauteur de la broche, par une surface oblique inclinée par rapport à l'axe médian longitudinal (3), tandis que le petit côté médial (13) débouche à la sortie du cône dans le col (6) de la prothèse par un arc ininterrompu à courbure constante, une saillie (12) en forme de collet séparant enfin les côtés (7) de la spatule et le col (6) de la prothèse, caractérisée par le fait que la saillie (12) en forme de collet, limitée aux côtés (7) de la spatule, s'étend au moins approximativement en direction du col (6) de la prothèse.

2. Prothèse coxo-fémorale selon la revendication 1, caractérisée par le fait que la saillie (12) en forme de collet s'étend en venant au moins approximativement à fleur de l'arête inférieure (8) du col de la prothèse.

*Fig.3*

*Fig.1*

*Fig.2*